(19) Europäisches Patentamt
European Patent Office
Office européen des brevets

(11) Veröffentlichungsnummer : **0 095 067**
**B1**

(12) # EUROPÄISCHE PATENTSCHRIFT

(45) Veröffentlichungstag der Patentschrift : **12.11.86**

(51) Int. Cl.⁴ : **C 07 D209/88**, A 61 K 31/40

(21) Anmeldenummer : **83104380.7**

(22) Anmeldetag : **04.05.83**

(54) **Verfahren zur Herstellung gegebenenfalls substituierter 1,2,3,4-Tetrahydro-9-cyanmethyl-carbazol-1-onen.**

(30) Priorität : **11.05.82 DE 3217563**

(43) Veröffentlichungstag der Anmeldung : **30.11.83 Patentblatt 83/48**

(45) Bekanntmachung des Hinweises auf die Patenterteilung : **12.11.86 Patentblatt 86/46**

(84) Benannte Vertragsstaaten : **AT BE CH DE FR GB IT LI NL SE**

(56) Entgegenhaltungen :
**DE-A- 2 114 230**
**US-A- 4 258 043**
**Die Akte enthält technische Angaben, die nach dem Eingang der Anmeldung eingereicht wurden und die nicht in dieser Patentschrift enthalten sind.**

(73) Patentinhaber : **CASSELLA Aktiengesellschaft**
**Hanauer Landstrasse 526**
**D-6000 Frankfurt am Main 61 (DE)**

(72) Erfinder : **Bender, Heinz, Dr.**
**Kirchgasse 20**
**D-6000 Frankfurt/Main 60 (DE)**
Erfinder : **Buch, Veit, Dr.**
**An der Pfingstweide 5**
**D-6368 Bad Vilbel (DE)**
Erfinder : **Beyerle, Rudi, Dr.**
**An der Pfaffenmauer**
**D-6000 Frankfurt/Main 60 (DE)**
Erfinder : **Kühlein, Klaus, Dr.**
**Fasanenstrasse 41**
**D-6233 Kelkheim (DE)**

(74) Vertreter : **Urbach, Hans-Georg, Dr. et al**
**Hanauer Landstrasse 526**
**D-6000 Frankfurt am Main 61 (DE)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

1,2,3,4-Tetrahydro-9-cyanmethyl-carbazol-1-one der allgemeinen Formel I

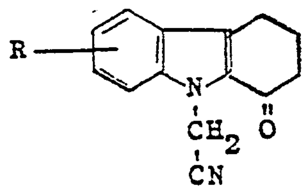

(I)

insbesondere solche, in denen R in der 6-Stellung des Carbazolsystems steht, sind wertvolle Zwischenprodukte zur Herstellung der z. B. aus der DE-OS-2 824 447 und der Europäischen Offenlegungsschrift 28270 bekannten psychotrop wirkenden Substanzen.

Aus der DE-OS-2 114 230 ist ein Verfahren zur Herstellung von 1,2,3,4-Tetrahydro-6-methyl-9-cyanmethyl-carbazol-1-on bekannt, bei dem man 1,2,3,4-Tetrahydro-6-methyl-carbazol-1-on in sein N-Metallderivat, z. B. in das N-Natriumderivat, überführt und dieses mit Chloracetonitril in einem organischen Lösungsmittel alkyliert. Bei diesem bekannten Verfahren wird zur Herstellung des Natriumsalzes eine Suspension von Natriumhydrid oder eine alkoholische Natriumalkoholatlösung zu der Lösung des 1,2,3,4-Tetrahydro-6-methyl-carbazol-1-ones in einem wasserfreien organischen Lösungsmittel, wie z. B. Dioxan, Dimethylformamid, Benzol, etc. gegeben. Im Fall der Verwendung von Natriumhydrid entwikkelt sich Wasserstoff; bei der Verwendung von Natriumalkoholaten wird der entsprechende Alkohol freigesetzt, fer abdestilliert werden muß. Nach der Bildung des Natriumderivats wird dem Reaktionsgemisch das Chloracetonitril zugesetzt. Bei diesem bekannten Verfahren werden nur dann nennenswerte Ausbeuten des gewünschten Endprodukts erhalten, wenn als organische Lösungsmittel dipolare aprotische Lösungsmittel, wie z. B. Dimethylformamid, eingesetzt werden und unter strengem Wasserausschluß gearbeitet wird. Dipolar aprotische Lösungsmittel, wie z. B. das genannte Dimethylformamid sind jedoch relativ teuer, sie sind schwer zu regenerieren, und sie dürfen auf keinen Fall in das Abwasser gelangen, um Umweltschäden zu vermeiden. Auch die Reinheit des nach dem bekannten Verfahren hergestellten Produkts läßt zu wünschen übrig. Will man es in der für die Weiterverarbeitung auf pharmazeutische Produkte erforderlichen Reinheit herstellen, so ist eine an die Cyanmethylierung anschließende Reinigungsoperation erforderlich, die nicht ohne Verluste durchgeführt werden kann. Bei kleineren Ansätzen werden daher nach dem bekannten Verfahren Ausbeuten von maximal 45 % d. Th. an Reinsubstanz erhalten, bei größeren Ansätzen, die eine längere Reaktionsdauer erfordern, sinken die Ausbeuten an Reinsubstanz auf 30 % d. Th. und darunter.

Aus der US-A-4 258 043 ist ein Verfahren zur Hestellung von substituierten 2,4,5,6-Tetrahydro-1H-pyrazino-(3,2,1-jk)-carbazolen bekannt, bei dem die erheblichen Schwierigkeiten, die bei dem aus der DE-A-2 114 230 bekannten Verfahren auftreten, vermieden werden. Bei diesem verbesserten Verfahren wird der kritische erste Schritt des Verfahrens der DE-A-2 114 230, die Cyanmethylierung des Carbazolderivats, bei welchem ein hochreaktives Cyanmethylierungsmittel eingesetzt werden muß, durch eine Halogenethylierung oder Hydroxyethylierung des Carbazolderivats, die mit einem wesentlich reaktionsträgeren Reagenz unter den Bedingungen einer Phasentransferreaktion ausgeführt werden kann, ersetzt.

Im Gegensatz zu allen dem Stand der Technik zu entnehmenden Erfahrungen und entgegen allen Erwartungen des Durchschnittsfachmannes wurde nun gefunden, daß man gegebenenfalls substituierte 1,2,3,4-Tetrahydro-9-cyanmethyl-carbazol-1-one der allgemeinen Formel

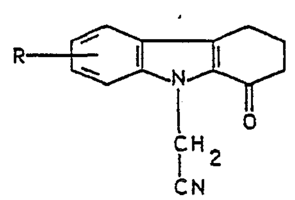

(I)

worin R Wasserstoff, Halogen, Alkyl oder Alkoxy bedeutet, in hoher Ausbeute und exzellenter Reinheit erhält, wenn man bei der Cyanmethylierung von gegebenenfalls substituierten 1,2,3,4-Tetrahydrocarbazol-1-onen der allgemeinen Formel II

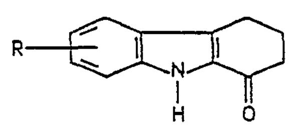

(II)

worin R die oben genannten Bedeutungen hat, das Ausgangsmaterial der Formel II mit einem Cyanmethylierungsmittel der allgemeinen Formel III

$$R^1—CH_2—CN \quad (III)$$

worin $R^1$ Halogen oder einen Rest der Formel $R^2—SO_2—O—$ bedeutet und $R^2$ für Alkyl, Phenyl oder substituiertes Phenyl steht, in einem Zweiphasensystem aus Wasser und einem mit Wasser nicht mischbaren organischen Lösungsmittel in Gegenwart einer starken Base und eines bekannten Phasentransferkatalysators umsetzt.

Die Reaktionsteilnehmer der erfindungsgemäßen Verfahrens, das Tetrahydrocarbazolderivat der Formel II, das Alkylierungsmittel der Formel III und die Base können grundsätzlich im Molverhältnis von 1 : 1 : 1 miteinander umgesetzt werden.

Um eine möglichst vollständige Cyanmethylierung des Carbazolderivats zu erreichen, wird jedoch zweckmäßigerweise ein Überschuß des Cyanmethylierungsmittels von 5 bis 30 mol%, vorzugsweise 10 bis 20 mol%, eingesetzt. Auch die bei der Cyanmethylierung anwesende Base wird in einem Überschuß von 2 bis 20fach, vorzugsweise 5 bis 10fach, bezogen auf Carbazolderivat, eingesetzt. Im Prinzip könnten Cyanalkylierungsmittel und Base auch in beliebig höherem Überschuß eingesetzt werden ; es werden jedoch damit keine wirtschaftlichen Vorteile mehr erzielt, da der geringe mögliche Zuwachs an Ausbeute den höheren Einsatz und die kompliziertere Aufarbeitung nicht mehr aufwiegt.

In den erfindungsgemäß herstellbaren gegebenenfalls substituierten 1,2,3,4-Tetrahydro-6-carbazol-1-onen der allgemeinen Formel I ist ein für R stehendes Halogen ein Fluor-, Chlor-, Brom- oder Jodatom, vorzugsweise ein Fluor-, Chlor- oder Bromatom.

Für R stehende Alkyl- oder Alkoxygruppen können geradkettig oder verzweigt sein und haben 1 bis 8, vorzugsweise 1 bis 4 C-Atome. Weiterhin ist die Herstellung von solchen 1,2,3,4-Tetrahydro-9-cyanmethyl-carbazol-1-onen nach dem erfindungsgemäßen Verfahren bevorzugt, bei denen R ungleich Wasserstoff ist, d. h. solchen, die im aromatischen Benzolkern des Tetrahydrocarbazol-Systems einen Substituenten aufweisen, insbesondere dann, wenn dieser Substituent in der 6-Stellung des Carbazolgerüstes steht.

In dem beim erfindungsgemäßen Verfahren eingesetzten Cyanmethylierungsmittel der Formel III bedeutet $R^1$ generell einen Rest, der in Gegenwart von Basen leicht als Anion abgespalten werden kann. Als besonders geeignet haben sich für $R^1$ Halogenatome, wie Chlor, Brom oder Jod oder Reste der Formel $R^2—SO_2—O—$, worin $R^2$ für Alkyl, Phenyl oder substituiertes Phenyl steht, erwiesen.

Besonders vorteilhaft ist der Einsatz von Cyanmethylierungsmitteln der allgemeinen Formel III, in denen $R^1$ Chlor, Brom oder ein Rest der Formel $R^2—SO_2—O—$ bedeutet, wobei $R^2$ für Alkyl mit 1 bis 5 C-Atomen, Phenyl oder durch Chlor oder Alkyl mit 1 bis 4 C-Atomen substituiertes Phenyl steht. Für das erfindungsgemäße Verfahren besonders bevorzugte Cyanmethylierungsmittel sind Chloracetonitril, Phenyl sulfonsäure-cyanmethylester und p-Toluolsulfonsäure-cyanmethylester.

Ein wesentliches Merkmal des erfindungsgemäßen Verfahrens ist die Durchführung der Reaktion in einem Zweiphasensystem aus Wasser und einem organischen Lösungsmittel. Als organische Lösungsmittel sind mit Wasser nicht mischbare Lösungsmittel geeignet, die sich unter den Reaktionsbedingungen inert verhalten, d. h. die nicht störend in die Reaktion eingreifen. Geeignete Lösungsmittel für das erfindungsgemäße Verfahren sind z. B. aliphatische, cycloaliphatische oder aromatische Kohlenwasserstoffe und aromatische Halogenkohlenwasserstoffe und höhersiedende Äther oder Polyäther soweit sie mit der wäßrigen, die Base gelöst enthaltenden Phase nicht mischbar sind. Beispiele für derartige Lösungsmittel sind Erdölfraktionen, wie z. B. Petrolether, Benzin, Ligroin oder Schwerbenzin, Cyclohexan, Benzol, Toluol, o-, m- oder p-Xylol bzw. deren technische Isomerengemische, Monochlor oder o-Dichlorbenzol, Dimethoxyethan. Auch Mischungen der genannten Lösungsmittel untereinander können verwendet werden. Besonders bevorzugt ist der Einsatz von Toluol oder technischen Xylol-Isomerengemischen.

Das Zweiphasensystem aus Wasser und einem mit Wasser nicht mischbaren organischen Lösungsmittel soll soviel organisches Lösungsmittel enthalten, daß durch die Reaktion der darin gelösten Anteile der Ausgangsmaterialien II und III eine ausreichend hohe Umsetzungsgeschwindigkeit und damit eine zufriedenstellende Reaktionsdauer für den Gesamtansatz erzielt wird. Zweckmäßigerweise wird soviel organisches Lösungsmittel eingesetzt, daß das Tetrahydrocarbazolon der Formel II zum größten Teil, vorzugsweise vollständig, gelöst wird. Die Wassermenge soll ausreichen, die zugesetzte Base zu lösen und gegebenenfalls entstehende Salze, z. B. Alkali- Benzol- bzw. Toluolsulfonat, weitgehend oder ganz in Lösung zu halten. In der Regel wird bis zur 30 fachen Gewichtsmenge, vorzugsweise 10 bis 20fachen Gewichtsmenge des Tetrahydrocarbazolons der Formel II an organischem Lösungsmittel eingesetzt. Das Gewichtsverhältnis von organischen Lösungsmittel und Wasser bewegt sich zwischen 1 : 0,2 und 1 : 2, vorzugsweise zwischen 1 : 0,4 und 1 : 0,7.

Die Cyanmethylierungsreaktion des erfindungsgemäßen Verfahrens wird in Gegenwart einer Base durchgeführt. Dieser Base kommt die Aufgabe zu, die Abspaltung des Restes $R^1$ aus dem Cyanmethylierungsmittel der Formel III als Anion einzuleiten und das bei der Reaktion freiwerdende Proton abzufangen. Geeignete Basen sind Verbindungen, die in wäßriger molarer Lösung einen pH-Wert von größer als 10 hervorrufen. Solche Basen sind beispielsweise Alkali- und bis zu einem gewissen Grad auch

Erdalkalisalze von schwachen und sehr schwachen anorganischen und organischen Säuren, wie zum Beispiel Alkalicarbonate, Trialkaliphosphate oder Alkaliborate und Alkali- und bis zu einem gewissen Grad auch Erdalkalihydroxide. Bevorzugt als Basen für das erfindungsgemäße Verfahren sind Alkalihydroxide, insbesondere Natrium- oder Kaliumhydroxid.

Ein weiteres Merkmal des erfindungsgemäßen Verfahrens ist der Einsatz von bekannten Phasentransferkatalysatoren. Eine zusammenfassende Übersicht über Phasentransferkatalyse- und -Katalysatoren befindet sich beispielsweise in dem Aufsatz von E. V. Dehmlow in « Angewandte Chemie » International Edition Engl. Band 13, S. 170 (1947).

Für die Durchführung des erfindungsgemäßen Verfahrens kommen als Phasentransferkatalysatoren insbesondere quartäre Ammonium- oder Phosphoniumsalze der Formeln IV und V in Betracht :

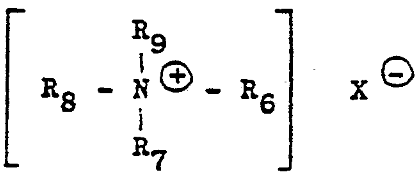
(IV)

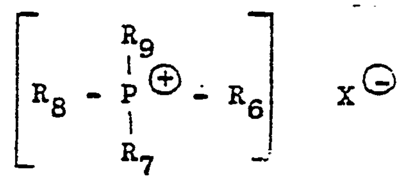
(V)

In diesen Formeln bedeuten $R_6$ bis $R_9$ Alkyl mit 1 bis 16 C-Atomen, Hydroxyalkyl mit 2 bis 16 C-Atomen, vorzugsweise 2 bis 4 C-Atomen, oder Alkoxyalkyl mit 1 bis 16 C-Atomen, vorzugsweise 2 bis 6 C-Atomen, Aryl, insbesondere Phenyl, Alkylphenyl mit 1 bis 10 C-Atomen, im Alkylrest, Aralkyl wie Benzyl oder Phenethyl und $X^{\ominus}$ die Ionen $F^{\ominus}$, $Cl^{\ominus}$, $Br^{\ominus}$, $SO_4^{\ominus\ominus}$, $HSO_4^{\ominus}$, $SO_3^{\ominus\ominus}$ und $HSO_3^{\ominus}$.

Für den erfindungsgemäßen Einsatzszweck besonders geeignete Phasentransferkatalysatoren sind beispielsweise Di-(-dodecyl)-dimethylammoniumchlorid, Hexadecyltrimethylammoniumchlorid, Tetrabutylammoniumchlorid, Trioctylmethylammoniumchlorid, Tris-decylmethylammoniumchlorid, Trialkyl-($C_8$-$C_{10}$-Gemisch)-methylammoniumchlorid, Tetrabutylammoniumhydrogensulfat, Benzyltrimethylammoniumchlorid und ähnliche Verbindungen. Kationen weiterer bekannter Phasentransferkatalysatoren sind z. B. Tetrapropylammonium, Tetradodecylammonium, Benzyltriethylammonium, Trihexylmethylammonium, Cetyltrimethylammonium, n-Alkyltriethylammonium, wobei der Alkylrest 4 oder mehr, beispielsweise 6 C-Atome, besitzt, Trioctylmethylammonium, Tricaprylylmethylammonium (Kation des Handelsprodukts Aliquat 336 der Fa. Gendval Mills. Comp., Kankakee, I11/USA), Hexadecyltributylphosphonium. Geeignete Anionen für diese Kationen sind insbesondere Hydrogensulfat, Chlorid und Bromid.

Bevorzugt für den erfindungsgemäßen Einsatz wird das Trialkyl-($C_8C_{10}$-Gemisch)-methylammoniumchlorid und Tetrabutylammoniumchlorid und Tetrabutylammoniumhydrogensulfat. Die Menge des einzusetzenden Phasentransferkatalysators beträgt zweckmäßigerweise 1 bis 10 mol%, bezogen auf die Menge des eingesetzten Carbazolons. Vorzugsweise werden 2 bis 6 mol% des Phasentransferkatalysators eingesetzt.

Die dem erfindungsgemäßen Verfahren zugrundeliegende Cyanmethylierungsreaktion verläuft unter den erfindungsgemäßen Verfahrensbedingungen bereits bei Raumtemperatur zügig und vollständig. Vorzugsweise wird daher das erfindungsgemäße Verfahren bei Temperaturen von 10 bis 30 °C ausgeführt. Is aus speziellen Gründen eine weitere Beschleunigung der Reaktion erwünscht, so kann dies in bekannter Weise durch Erwärmen des Reaktionsansatzes erfolgen. In diesem Fall wird die Reaktionstemperatur zweckmäßigerweise unterhalb der Siedetemperatur des mit Wasser nicht mischbaren organischen Lösungsmittels gewählt. Eine Möglichkeit besteht darin, beim Siedepunkt des Azeotrops von organischem Lösungsmittel und Wasser zu arbeiten. Normalerweise wird eine ausreichende Beschleunigung der Reaktion bereits beim Anwärmen auf max. 50 °C erreicht. Die Reaktion kann auch noch unterhalb von 10 °C mit guten Ausbeuten durchgeführt werden, wenn eine entsprechend längere Reaktionszeit in Kauf genommen wird.

Das erfindungsgemäße Verfahren kann in der Weise ausgeführt werden, daß die Reaktionsteilnehmer, das Tetrahydrocarbazolon der Formel II, das Cyanmethylierungsmittel der Formel III und die Base in beliebiger Reihenfolge vollständig in das vorgelegte Lösungsmittelsystem aus Wasser und organischem Lösungsmittel eingetragen werden zweckmäßigerweise unter stotiger Durchmischung und der Ansatz bis zum Abschluß der Reaktion unter Durchmischung bei der gewünschten Temperatur gehalten wird. Vorteilhaft im Hinblick auf Verseifungsreaktionen kann es sein, das Cyanmethylierungsmittel oder die Base oder beide Komponenten dem Reaktionsansatz nach und nach portionsweise oder kontinuierlich zuzufügen.

Bei Einsatz von Phenyl- bzw. Tolylsulfonsäure-cyanmethylester als Cyanmethylierungsmittel ist eine besonders vorteilhafte Ausführungsform des erfindungsgemäßen Verfahrens durchführbar. Diese besteht darin, daß man die genannten Cyanmethylierungsmittel nicht in isolierter, reiner Form einsetzt, sondern in Form einer bei ihrer Herstellung anfallenden rohen Dispersion. Diese Variante ist auch als « Eintopfreaktion » durchführbar, indem zunächst in einem hinreichend großen Reaktionsgefäß aus Benzol-, bzw. p-Toluolsulfochlorid, Formaldehyd und Alkalicyanid in wäßrigem Medium in bekannter Weise (vergl. z. B. Lichtenberger, Bull. Soc. Chim. France (*1948*) Seiten 995-1001) eine Dispersion eines

der genannten Cyanmethylester hergestellt wird, und, ohne Isolierung dieser Verbindung, der Dispersion eine Lösung des 1,2,3,4-Tetrahydrocarbazol-1-on-Derivats der Formel II in dem organischen Lösungsmittel, vorzugsweise Toluol oder Xylol, zugesetzt wird. Nach Zugabe des Phasentransferkatalysators wird die Reaktion unter Eintropfen der wäßrigen Lösung der Base, vorzugsweise von verdünnter Natronlauge, durchgeführt. Außer der Zeit- und Materialersparnis hat diese Arbeitsweise den Vorteil, daß jeder Kontakt des Betriebspersonals mit den gesundheitlich nicht ganz unbedenklichen Alkylierungsmitteln der Formel III vermieden wird.

Nach dem Abschluß der Cyanmethylierungsreaktion wird ein evtl. noch vorhandener Überschuß des Cyanmethylierungsmittels der Formel III durch den im System vorhandenen Basenüberschuß verseift.

Die Isolierung der erfindungsgemäß hergestellten Verbindungen der Formel I erfolgt in an sich bekannter Weise.

Sofern bei Verwendung einer Verhältnismäßig geringen Menge an organischem Lösungsmittel eine Abscheidung des Endprodukts erfolgt, kann dieses direkt abfiltriert und durch Waschen mit Wasser von Basenüberschuß und Salzen befreit werden.

Hat man so viel organisches Lösungsmittel eingesetzt, daß die organischen Verbindungen vollständig gelöst werden, so trennt man beispielsweise nach Abschluß der Reaktion die organische von der wäßrigen Phase ab und engt sie, vorteilhafterweise unter vermindertem Druck, bis zur Abscheidung der Endprodukts ein. Dem Einengen können selbstverständlich, wenn erforderlich, eine Schmutzfiltration, Wasch- oder/und Trockenoperationen oder andere bekannte Aufarbeitungsoperationen vorgeschaltet werden. Selbstverständlich können auch andere bekannte Wege zur Isolierung des Endprodukts beschritten werden. So kann nach einer groben Phasentrennung die organische Phase destilliert werden, wobei das darin noch enthaltene Wasser azeotrop abgetrieben wird. Wenn das Wasser und die erforderliche Menge des organischen Lösungsmittels abdestilliert sind, kann eine Schmutzfiltration erfolgen. Durch Abkühlen des Filtrats läßt sich das Auskristallisieren den Endprodukts herbeiführen. Die Produkte fallen bei dieser Arbeitsweise in der Regel so rein an, daß weitere Reinigungsoperationen überflüssig werden.

Die folgenden Ausführungsbeispiele veranschaulichen das erfindungsgemäße Verfahren :

## Beispiel 1

Zu einer gemäß Lichtenberger, Bul. Soc. Chim. France (1948) Seite 998 aus Benzolsulfochlorid, Formaldehyd und Natriumcyanid in 230 ml Wasser hergestellten Suspension von 1,1 mol Cyanmethylbenzolsulfonat gibt man 4 000 ml Toluol, 20 g Tetrabutylammoniumhydrogensulfat und 199 g (1 mol) 1,2,3,4-Tetrahydro-6-methylcarbazol-1-on. Unter kräftigem Rühren tropft man 1 180 ml Natronlauge (10 mol) bei 20-25 °C innerhalb von 10-20 Min. ein. Nach 1 Std. wird vom ausgefallenen Natriumbenzolsulfonat abgesaugt. Das Filtrat wird absitzen gelassen und organische und wäßrige Phase werden im Scheidetrichter voneinander getrennt. Die Toluolschicht wird mit saurem Wasser gewaschen, eingedampft und der Rückstand aus Ethanol umkristallisiert. Ausbeute : 214 g = 90 % d. Th., Schmelzpunkt 140°.

## Beispiel 2

199 g (1 mol) 1,2,3,4-Tetrahydro-6-methyl-carbazol-1-on, 4 000 ml Toluol, 1 180 ml Natronlauge (10 mol) und 20 g Tetrabutylammoniumhydrogensulfat werden bei 20-25 °C vorgelegt und 30 Min. gerührt. Innerhalb von 2 Std. werden dann 91 g (1,2 mol) Chloracetonitril bei 20-25 °C zugetropft und es wird anschließend noch 10 Min. nachgerührt. Nach dem Absitzen und der Phasentrennung wird die Toluolschicht mit sauren Wasser gewaschen und eingedampft. Der Rückstand wird aus Ethanol umkristallisiert.

Die Ausbeute an 1,2,3,4-Tetrahydro-6-methyl-9-cyanmethylcarbazol-1-on beträgt 190 g (~ 80 % d. Th.), Schmelzpunkt 138-139 °C.

## Beispiel 3

Zu einer gemäß Lichtenberger, Bul. Soc. Chim. France (1948) Seite 998 aus Benzolsulfochlorid, Formaldehyd und Natriumcyanid in 230 ml Wasser hergestellten Suspension von 1,1 mol Cyanmethylbenzolsulfonat gibt man 4 000 ml technisches Xylolgemisch, 20 g Tetrabutylammoniumhydrogensulfat und 199 g (1 mol) 1,2,3,4-Tetrahydro-6-methylcarbazol-1-on. Unter kräftigem Rühren tropft man 1 180 ml Kalilauge (10 mol) bei 20-25 °C innerhalb von 10-20 Min. ein. Nach 1 Std. wird vom ausgefallenen Natriumbenzolsulfonat abgesaugt. Das Filtrat wird absitzen gelassen und organische und wäßrige Phase werden im Scheidetrichter voneinander getrennt. Die Toluolschicht wird mit saurem Wasser gewaschen, eingedampft und der Rückstand aus Ethanol umkristallisiert.

Ausbeute : 210 g = 88 % d. Th.,

Schmelzpunkt 140 °C.

Wird die Reaktion nicht bei 20-25 °C, sondern bei 50 °C durchgeführt, so kann das Absaugen des Natriumbenzolsulfonats bereits nach 1/2 Std. erfolgen und man erhält 212 g Ausbeute, entsprechend

89 % der Theorie. Ersetzt man das oben eingesetzte technische Xylol durch die gleiche Menge Monochlorbenzol so erhält man die gleiche Ausbeute.

Mit anderen Lösungsmitteln werden folgende Ausbeuten erhalten :

| | |
|---|---|
| Ligroin (Erdölfraktion Kp ~ 150 °C) | 87 % d. Th. |
| Cyclohexan | 86 % d. Th. |
| 1,2-Dimethoxy-ethan | 81 % d. Th. |

Beispiel 4

199 g (1 mol) 1,2,3,4-Tetrahydro-6-methyl-carbazol-1-on, 4 000 ml Toluol, 1 180 ml Natronlauge (10 ml) und 20 g Tetrabutylammoniumhydrogensulfat werden bei 5-10 °C vorgelegt und 30 Min. gerührt. Innerhalb von 2 Std. werden dann 91 g (1,2 mol) Chloracetonitril bei 5-10 °C zugetropft und anschließend noch 1-2 Std. nachgerührt. Nach dem Absitzen und der Phasentrennung wird die Toluolschicht mit saurem Wasser gewaschen und eingedampft. Der Rückstand wird aus Ethanol umkristallisiert.

Die Ausbeute an 1,2,3,4-Tetrahydron-6-methyl-9-cyanmethylcarbazol-1-on beträgt 198 g (~ 83,4 % d. Th.), Schmelzpunkt 139 °C.

Beispiel 5

Zu einer gemäß Lichtenberger, Bul. Soc. Chim. France (1948) Seite 998 aus Benzolsulfochlorid, Formaldehyd und Natriumcyanid in 230 ml Wasser hergestellten Suspension von 1,1 mol Cyanmethylbenzolsulfonat gibt man 4 000 ml Toluol, 20 g Tricapryl-methylammoniumchlorid und 199 g (1 mol) 1,2,3,4-Tetrahydro-6-methylcarbazol-1-on. Unter kräftigem Rühren tropft man 1 180 ml Natronlauge (10 mol) bei 20-25 °C innerhalb von 10-20 Min. ein. Nach 1 Std. wird vom ausgefallenen Natriumbenzolsulfonat abgesaugt. Das Filtrat wird absitzen gelassen und organische und wäßrige Phase werden im Scheidetrichter voneinander getrennt. Die Toluol-Schicht wird mit saurem Wasser gewaschen, eingedampft und der Rückstand aus Ethanol umkristallisiert.

Ausbeute : 217,5 g = 91,5 % d. Th., Schmelzpunkt 140 °C.

Vermindert man im obigen Beispiel die Menge des Cyanmethylbenzolsulfonats auf 1,0 mol, so erhält man eine Ausbeute von 84 % d. Th.

Bei Ensatz von 1,5 mol Cyanmethylbenzolsulfonat steigt die Ausbeute auf 91 % d. Th., bei Einsatz von 2,5 mol auf 91,5 % d. Th.

Eine weitere Steigerung auf 4 mol Cyanmethylbenzolsulfonat führt zu einer Ausbeute von 92 % d. Th.

Variiert man in obigem Beispiel den Phasentransferkatalysator, so werden folgende Ausbeuten erhalten :

| | |
|---|---|
| Hexadecyl-trimethyl-ammoniumchlorid : | 90 % d. Th. |
| Trisdecyl-methyl-ammoniumchlorid : | 88 % d. Th. |
| Benzyl-trimethyl-ammoniumchlorid : | 89 % d. Th. |
| Benzyl-triethylammonium-hydrogensulfat : | 89 % d. Th. |
| Hexadecyl-trimethylphosphonium-hydrogensulfat : | 91 % d. Th. |
| Hexadecyl-tibutylphosphonium-chlorid : | 89 % d. Th. |

Beispiel 6

Zu einer gemäß Lichtenberger, Bul. Soc. Chim. France (1948) Seite 998 aus Benzolsulfochlorid, Formaldehyd und Natriumcyanid in 230 ml Wasser hergestellten Suspension von 1,1 mol Cyanmethylbenzolsulfonat gibt man 4 000 ml Benzol, 20 g Tetrabutylammoniumhydrogensulfat und 199 g (1 mol) 1,2,3,4-Tetrahydro-6-methylcarbazol-1-on. Die Mischung wird zum Siedem erwärmt und bei schwachem Rückfluß und kräftigem Rühren läßt man .innerhalb von 10 Minuten 1 380 ml einer 50 Gew. %igen wäßrigen Kaliumcarbonatlösung (5 mol) einlaufen.

Nach 1 Std. wird unter einem gut ziehenden Abzug vom ausgefallenen Natriumbenzolsulfonat abgesaugt. Das Filtrat wird absitzen gelassen und organische und wäßrige Phase werden im Scheidetrichter voneinander getrennt. Die Benzolschicht wird mit saurem Wasser gewaschen, eingedampft und der Rückstand aus Ethanol umkristallisiert. Ausbeute : 191 g ~ 80,3 % d. Th., Schmelzpunkt 140 °C. Ersetzt man in obiger Vorschrift das Benzol durch Petroläther (Erdölfraktion vom Kp 65-75 °C), so wird eine Ausbeute von 197,4 g ~ 83 % d. Th. erhalten.

Beispiel 7

199 g (1 mol) 1,2,3,4-Tetrahydro-6-methyl-carbazol-1-on, 4 000 ml Toluol, eine 50 °C warme Lösung von 256 g Bariumhydroxyd in 2 300 ml destilliertem, kohlensäurefreiem Wasser (1,5 mol) und 20 g Tetrabutylammoniumchlorid werden bei 20-25 °C vorgelegt, die Luft aus dem Reaktionsgefäß durch Stickstoff verdrängt und unter $CO_2$-Ausschluß 30 Min. gerührt. Innerhalb von 2 Std. werden dann 91 g

(1,2 mol) Chloracetonitril bei 40-50 °C zugetropft und anschließend noch 30 Min. nachgerührt. Nach dem Absitzen und der Phasen trennung wird die Toluolschicht mit saurem Wasser gewaschen und eingedampft. Der Rückstand wird aus Ethanol umkristallisiert.

Die Ausbeute an 1,2,3,4-Tetrahydro-6-methyl-9-cyanmethyl-carbazol-1-on beträgt 195 g (~ 82 % d. Th.), Schmelzpunkt 138-139 °C.

Wird anstelle von Bariumhydroxyd die äquivalente Menge Lithiumhydroxyd eingesetzt, so erhält man eine Ausbeute von 86 % d. Th.

Setzt man anstelle des in den Beispielen 1 und 2 eingesetzten 1,2,3,4-Tetrahydro-6-methyl-carbazol-1-ons als Ausgangsmaterial äquivalente Mengen anderer, in 6-Stellung durch Chlor, Brom, Fluor oder Methoxy substituierter 1,2,3,4-Tetrahydrocarbazol-1-one der allgemeinen Formel II ein, und arbeitet im übrigen wie in den Beispielen angegeben, so erhält man in ebenfalls sehr guten Ausbeuten :

1,2,3,4-Tetrahydro-6-chlor-9-cyanmethyl-carbazol-1-on Fp. : 159 °C.
1,2,3,4-Tetrahydro-6-brom-9-cyanmethyl-carbazol-1-on Fp. : 180-181 °C.
1,2,3,4-Tetrahydro-6-fluor-9-cyanmethyl-carbazol-1-on Fp. : 136 °C.
1,2,3,4-Tetrahydro-6-methoxy-9-cyanmethyl-carbazol-1-on Fp. : 138 °C.

**Patentansprüche**

1. Verfahren zur Herstellung gegebenenfalls substituierter 1,2,3,4-Tetrahydro-9-cyanmethyl-carbazol-1-onen der allgemeinen Formel I

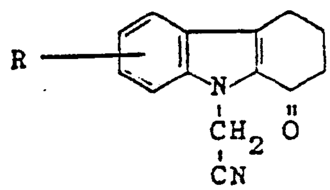

(I)

worin R Wasserstoff, Halogen, Alkyl oder Alkoxy bedeutet durch Cyanmethylierung von gegebenenfalls substituierten 1,2,3,4-Tetrahydro-carbazol-1-onen der allgemeinen Formel II

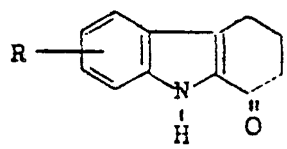

(II)

worin R die oben genannten Bedeutungen hat, dadurch gekennzeichnet, daß man das Ausgangsmaterial der Formel II mit einm Cyanmethylierungsmittel der allgemeinen Formel III

$$R^1—CH_2—CN \qquad (III)$$

worin $R^1$ Halogen oder einen Rest der Formel $R^2—SO_2—O—$ bedeutet und $R^2$ für Alkyl, Phenyl oder substituiertes Phenyl steht, in einem Zweiphasensystem aus Wasser und einem mit Wasser nicht mischbaren organischen Lösungsmittel in Gegenwart einer starken Base und eines bekannten Phasentransferkatalysators umsetzt.

2. Verfahren gemäß Anspruch 1 zur Herstellung von 1,2,3,4-Tetrahydro-9-cyanmethyl-carbazol-1-onen der allgemeinen Formel I, worin R Wasserstoff, Fluor, Chlor, Brom, Alkyl mit 1 bis 8 C-Atomen oder Alkoxy mit 1 bis 8 C-Atomen bedeutet, dadurch gegenzeichnet, daß ein 1,2,3,4-Tetrahydro-carbazol-1-on der allgemeinen Formel II eingesetzt wird, in dem R die hier genannten Bedeutungen hat.

3. Verfahren gemäß den Ansprüchen 1 und 2 zur Herstellung von 1,2,3,4-Tetrahydro-9-cyanmethyl-carbazol-1-onen der allgemeinen Formel I, worin R Wasserstoff, Fluor, Chlor, Brom, Alkyl mit 1 bis 4 C-Atomen oder Alkoxy mit 1 bis 4 C-Atomen bedeutet, dadurch gekennzeichnet, daß ein 1,2,3,4-Tetrahydro-carbazol-1-on der allgemeinen Formel II eingesetzt wird, in dem R die hier genannten Bedeutungen hat.

4. Verfahren gemäß den Ansprüchen 1 bis 3 zur Herstellung von 1,2,3,4-Tetrahydro-9-cyanmethyl-carbazol-1-onen der allgemeinen Formel I, worin R ungleich Wasserstoff ist.

5. Verfahren gemäß Anspruch 4, dadurch gekennzeichnet, daß R in der 6-Stellung des Carbazolsystems steht.

6. Verfahren gemäß den Ansprüchen 1 bis 5, dadurch gekennzeichnet, daß ein Cyanmethylierungsmittel der allgemeinen Formel III eingesetzt wird, worin $R^1$ Chlor, Brom, Jod oder einen Rest der Formel $R^2—SO_2—O—$ bedeutet und $R^2$ für Alkyl mit 1 bis 18 C-Atomen, Phenyl oder durch Halogen, Niederalkyl

oder Niederalkoxy substituiertes Phenyl steht.

7. Verfharen gemäß Anspruch 6, dadurch gekenzeichnet, daß ein Cyanmethylierungsmittel der allgemeinen Formel III eingesetzt wird, worin $R^1$ Chlor, Brom oder einen Rest der Formel $R^2—SO_2—O—$ bedeutet und $R^2$ für Alkyl mit 1 bis 5 C-Atomen, Phenyl oder durch Chlor oder Niederalkyl substituiertes Phenyl steht.

8. Verfahren gemäß Anspruch 1, dadurch gekennzeichnet, daß ein Cyanmethylierungsmittel der allgemeinen Formel III, worin $R^1$ einen Rest der Formel $R^2—SO_2—O—$ bedeutet und $R^2$ für Phenyl oder durch Niederalkyl substituiertes Phenyl steht, in Form einer bei seiner Herstellung anfallenden, rohen Dispersion, ohne Zwischenisolierung, eingesetzt wird.

**Claims**

1. Process for the preparation of optionally substituted 1,2,3,4-tetrahydro-9-cyanomethylcarbazol-1-ones of the general formula I

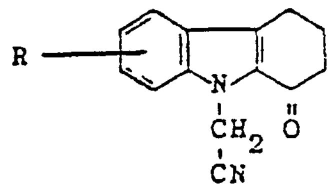

(I)

wherein R denotes hydrogen, halogen, alkyl or alkoxy, by cyanomethylating optionally substituted 1,2,3,4-tetrahydrocarbazol-1-ones of the general formula II

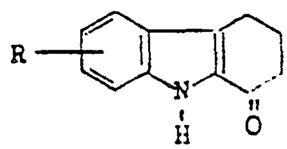

(II)

wherein R has the meanings mentioned above, characterised in that the starting material of the formula II is reacted, in a two-phase system consisting of water and a water-immiscible organic solvent, in the presence of a strong base and a known phase transfer catalyst, with a cyanomethylating agent of the general formula III

$$R^1—CH_2—CN \quad \text{(III)}$$

wherein $R^1$ denotes halogen or a radical of the formula $R^2—SO_2—O—$ and $R^2$ represents alkyl, phenyl or substituted phenyl.

2. Process according to Claim 1 for the preparation of 1,2,3,4-tetrahydro-9-cyanomethylcarbazol-1-ones of the general formula I wherein R denotes hydrogen, fluorine, chlorine, bromine, alkyl having 1 to 8 C atoms or alkoxy having 1 to 8 C atoms, characterised in that a 1,2,3,4-tetrahydrocarbazol-1-one of the general formula II in which R has the meanings mentioned herein, is employed.

3. Process according to Claims 1 and 2 for the preparation of 1,2,3,4-tetrahydro-9-cyanomethylcarbazol-1-ones of the general formula I wherein R denotes hydrogen, fluorine, chlorine, bromine, alkyl having 1 to 4 C atoms or alkoxy having 1 to 4 C atoms, characterised in that a 1,2,3,4-tetrahydrocarbazol-1-one of the general formula II in which R has the meanings mentioned herein, is employed.

4. Process according to Claims 1 to 3 for the preparation of 1,2,3,4-tetrahydro-9-cyanomethylcarbazol-1-ones of the general formula I wherein R is not hydrogen.

5. Process according to Claim 4, characterised in that R is in the 6-position of the carbazole system.

6. Process according to Claims 1 to 5, characterised in that a cyanomethylating agent of the general formula III wherein $R^1$ denotes chlorine, bromine, iodine or a radical of the formula $R^2—SO_2—O—$ and $R^2$ represents alkyl having 1 to 18 C atoms, phenyl or phenyl which is substituted by halogen, lower alkyl or lower alkoxy, is employed.

7. Process according to Claim 6, characterised in that a cyanomethylating agent of the general formula III wherein $R^1$ denotes chlorine, bromine or a radical of the formula $R^2—SO_2—O—$ and $R^2$ represents alkyl having 1 to 5 C atoms, phenyl or phenyl which is substituted by chlorine or lower alkyl, is employed.

8. Process according to Claim 1, characterised in that a cyanomethylating agent of the general formula III wherein $R^1$ denotes a radical of the formula $R^2—SO_2—O—$ and $R^2$ represents phenyl or phenyl which is substituted by lower alkyl, is employed, without intermediate isolation, in the form of a crude

dispersion produced in the preparation thereof.

**Revendications**

1. Procédé de préparation de tétrahydro-1,2,3,4 cyanométhyl-9 carbazolones-1, éventuellement substituées, qui répondent à la formule générale I

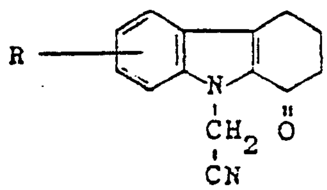

(I)

dans laquelle R représente l'hydrogène, un halogène, un alkyle ou un alcoxy, par cyanométhylation de tétrahydro-1,2,3,4 carbazolones-1, éventuellement substituées, répondant à la formule II

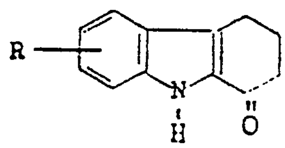

(II)

dans laquelle R a la signification précédemment donnée, procédé caractérisé en ce qu'on fait réagir le corps de départ de formule II avec un agent de cyanométhylation répondant à la formule générale III

$$R^1—CH_2—CN \quad (III)$$

dans laquelle $R^1$ représente un halogène ou un radical $R^2—SO_2—O—$ dans lequel $R^2$ représente un alkyle, un phényle ou un phényle substitué, dans un système diphasique constitué d'eau et d'un solvant organique non miscible à l'eau, en présence d'une base forte et d'un catalyseur de transfert de phase connu.

2. Procédé selon la revendication 1 pour la préparation de tétrahydro-1,2,3,4 cyanométhyl-9 carbazolones-1 de formule générale I dans lesquelles R représente l'hydrogène, le fluor, le chlore, le brome, un alkyle contenant de 1 à 8 atomes de carbone ou un alcoxy contenant de 1 à 8 atomes de carbone, procédé caractérisé en ce qu'on utilise une tétrahydro-1,2,3,4 carbazolone-1 de formule générale II dans laquelle R a la signification qui vient d'être indiquée.

3. Procédé selon l'une des revendications 1 et 2 pour la préparation de tétrahydro-1,2,3,4 cyanométhyl-9 carbazolones-1 de formule générale I dans lesquelles R représente l'hydrogène, le fluor, le chlore, le brome, un alkyle contenant de 1 à 4 atomes de carbone ou un alcoxy contenant de 1 à 4 atomes de carbone, procédé caractérisé en ce qu'on utilise une tétrahydro-1,2,3,4 carbazolone-1 de formule générale II dans laquelle R a la signification qui vient d'être indiquée.

4. Procédé selon l'une quelconque des revendications 1 à 3 pour la préparation de tétrahydro-1,2,3,4 cyanométhyl-9 carbazolones-1 de formule générale I dans lesquelles R n'est pas l'hydrogène.

5. Procédé selon la revendication 4 caractérisé en ce que R se trouve en position 6 sur le système du carbazole.

6. Procédé selon l'une quelconque des revendications 1 à 5, caractérisé en ce qu'on utilise un agent de cyanométhylation de formule générale III dans lequel $R^1$ représente le chlore, le brome, l'iode ou un radical de formule $R^2—SO_2—O—$ et $R^2$ représente un alkyle contenant de 1 à 18 atomes de carbone, un phényle ou un phényle porteur d'un halogène, d'un alkyle inférieur ou d'un alcoxy inférieur.

7. Procédé selon la revendication 6 caractérisé en ce qu'on utilise un agent de cyanométhylation de formule générale III dans lequel $R^1$ représente le chlore, le brome ou un radical de formule $R^2—SO_2—O—$ et $R^2$ représente un alkyle contenant de 1 à 5 atomes de carbone, un phényle ou un phényle porteur d'un atome de chlore et d'un alkyle inférieur.

8. Procédé selon la revendication 1 caractérisé en ce qu'on utilise un agent de cyanométhylation de formule générale III dans lequel $R^1$ représente un radical de formule $R^2—SO_2—O—$ et $R^2$ représente un radical phényle ou un radical phényle porteur d'un alkyle inférieur, sous la forme d'une dispersion brute obtenue lors de sa préparation, sans isolement intermédiaire.